Europäisches Patentamt

**European Patent Office**

**Office européen des brevets**

(19)

(11) Numéro de publication: **0 142 422**

**B1**

(12) **FASCICULE DE BREVET EUROPÉEN**

(45) Date de publication du fascicule du brevet:
**27.12.89**

(51) Int. Cl.⁴: **G 01 N 15/04,** G 01 N 13/00

(21) Numéro de dépôt: **84402150.1**

(22) Date de dépôt: **24.10.84**

(54) **Compteur de phases liquides d'un mélange partiellement liquide et transparent.**

(30) Priorité: **25.10.83 FR 8316973**

(43) Date de publication de la demande:
**22.05.85 Bulletin 85/21**

(45) Mention de la délivrance du brevet:
**27.12.89 Bulletin 89/52**

(84) Etats contractants désignés:
**DE GB**

(56) Documents cité:
**DE-A-2 551 260**
**DE-A-3 203 882**
**GB-A-2 017 936**
**US-A-2 971 371**
**US-A-2 982 170**

(73) Titulaire: **CENTRE NATIONAL DE LA RECHERCHE SCIENTIFIQUE (CNRS), 15, Quai Anatole France, F-75700 Paris Cedex 07 (FR)**

(72) Inventeur: **Bothorel, Pierre, 132, Avenue du Docteur Schweitzer, F-33600 Pessac (FR)**
Inventeur: **Bernon, Roland, 1, Chemin de Cantelaude, F-33610 Cestas (FR)**
Inventeur: **Gabriel, Guy, 26 Bis, rue Phénix Haut-Brion, F-33600 Pessac (FR)**

(74) Mandataire: **Mongrédien, André, c/o BREVATOME 25, rue de Ponthieu, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet un compteur de phases liquides. Ce compteur permet de construire le diagramme des phases d'un mélange partiellement liquide et transparent. Ces diagrammes sont utilisés notamment dans l'industrie pétrolière, dans le traitement des minerais en milieu aqueux, en cosmétologie,...

Dans le domaine pétrolier par exemple, le compteur de l'invention trouve une application en récupération assistée du pétrole. Depuis le premier choc pétrolier en 1973, les compagnies pétrolières ont essayé d'améliorer le rendement d'extraction du pétrole, qui est actuellement limité à 35 % pour des raisons techniques et économiques. Le procédé utilisé consiste à injecter dans le puits de pétrole une micro-émulsion monophasique qui devient, lors du balayage du gisement, un système triphasique. Cette micro-émulsion est constituée d'un mélange contenant au moins un agent tensioactif, de l'eau, pure ou salée, un hydrocarbure et éventuellement un agent cotensioactif, par exemple un alcool. Selon les proportions de chacun des constituants de ce mélange, le système peut être, à l'équilibre, mono, di, tri ou tétraphasique. Il faut donc effectuer un tri parmi les mélanges pour ne garder que ceux conduisant à un système triphasique optimisé, seul utilisable en récupération assistée du pétrole.

On ne connaît pas de méthode théorique permettant de construire a priori le diagramme des phases et donc déterminer les mélanges intéressants. Seule l'étude expérimentale d'un grand nombre de mélanges permet de sélectionner les mélanges utiles, c'est-à-dire triphasiques à l'équilibre.

Selon l'art antérieur, la construction du diagramme des phases d'un mélange est réalisée manuellement. Pour que ce diagramme soit précis, il faut relever le nombre de phases d'un grand nombre de points. Ce relevé est très long. A titre d'exemple, le relevé de 15 000 points de ce diagramme nécessite plusieurs années de travail ininterrompu. Outre la lenteur, ce procédé de construction d'un diagramme des phases est relativement peu précis, la composition de chaque mélange, préparé manuellement, étant elle-même de précision limitée.

On pourra se reporter par ailleurs à l'état de la technique constitué par le brevet US-A-2 982 170 dans lequel il est décrit un compteur de phases liquides d'un mélange partiellement liquide et transparent, le compteur comprenant:

- au moins une cellule fixée sur un support, chaque cellule étant disposée verticalement et contenant un mélange partiellement liquide et transparent,
- un moyen optique de mesure de la transparence optique du mélange entre deux parois d'une cellule,
- un moyen de déplacement, pour déplacer le moyen de mesure optique sur toute la hauteur d'une cellule parallèlement à celle-ci, et un moyen de positionnement, pour positionner ledit moyen au droit de chaque cellule,
- un moyen électronique de commande et de traitement délivrant des signaux pour commander les moyens de déplacement et de positionnement et apte à déterminer le nombre de phases d'un mélange au vu des signaux reçus du moyen optique de mesure. En outre, dans la demande de brevet DE-A-2 551 260 on trouve une description de moyens d'homogénéisation. Toutefois, cet état de la technique ne permet pas d'avoir une entière automatisation et donc de compléter un mélange partiel initial en ajoutant le ou les derniers constituants liquides.

L'invention a pour but de remédier à ces inconvénients. Elle a pour objet un compteur de phases liquides capable de compléter un mélange partiel initial en ajoutant le ou les derniers constituants liquides, d'assurer l'homogénéisation par agitation, d'observer si l'équilibre thermodynamique est atteint, et de compter le nombre de phases. Ce compteur peut traiter en même temps plusieurs mélanges, chacun d'eux étant dilué automatiquement au cours du temps.

De façon plus précise, le compteur de phases liquides comprend:

- au moins une cellule fixée sur un support, chaque cellule étant disposée verticalement et contenant un mélange partiellement liquide et transparent,
- un moyen d'homogénéisation du mélange de chaque cellule,
- un moyen optique de mesure de la transparence optique du mélange entre deux parois d'une cellule,
- un moyen de déplacement pour déplacer le moyen d'homogénéisation et le moyen de mesure sur toute la hauteur d'une cellule parallèlement à celle-ci, et un moyen de positionnement pour positionner lesdits moyens au droit de chaque cellule,
- un moyen électronique de commande et de traitement délivrant des signaux pour commander les moyens de déplacement et de positionnement et le moyen d'homogénéisation et apte à déterminer le nombre de phases d'un mélange au vu des signaux reçus du moyen optique de mesure, il comprend en outre pour chaque cellule et pour effectuer un relevé du diagramme des phases d'un mélange partiellement liquide et transparent, au moins une source d'un constituant liquide du mélange, une pompe volumétrique par source et des électrovannes reliant ladite pompe à chaque cellule, ladite pompe et les électrovannes étant commandées par le moyen électronique.

Le moyen de positionnement n'est utile que dans le cas d'un compteur à au moins deux cellules. Selon une caractéristique secondaire, les cellules sont disposées sur une couronne circulaire dont le centre constitue l'axe du moyen de positionnement.

Selon une autre caractéristique secondaire, le moyen de mesure optique comprend une source lumineuse et une photodiode en regard disposées de part et d'autre de la cellule.

Selon une autre caractéristique secondaire, le moyen d'homogénéisation comprend un électroaimant et un barreau magnétique.

Selon une autre caractéristique secondaire, un aimant permanent est disposé dans la cellule ou à l'extérieur de celle-ci, au-dessus du mélange, constituant une position de repos pour le barreau magnétique.

Les caractéristiques et avantages de l'invention ressortiront mieux de la description qui va suivre, donnée à titre illustratif mais non limitatif, en référence aux figures annexées, dans lesquelles:

- la figure 1 représente un mode de réalisation d'un compteur de phases liquides à une seule cellule selon l'invention,
- la figure 2 représente une variante du compteur selon l'invention,
- la figure 3a représente une coupe du moyen d'homogénéisation en vue de dessus,
- la figure 3b représente une coupe du moyen de mesure optique en vue de dessus,
- la figure 4 représente le signal délivré par le capteur optique correspondant au mélange contenu dans la cellule,
- la figure 5 représente un diagramme des phases obtenu par le compteur de phases liquides de l'invention,
- la figure 6 représente un compteur de phases liquides selon l'invention, à plusieurs cellules.

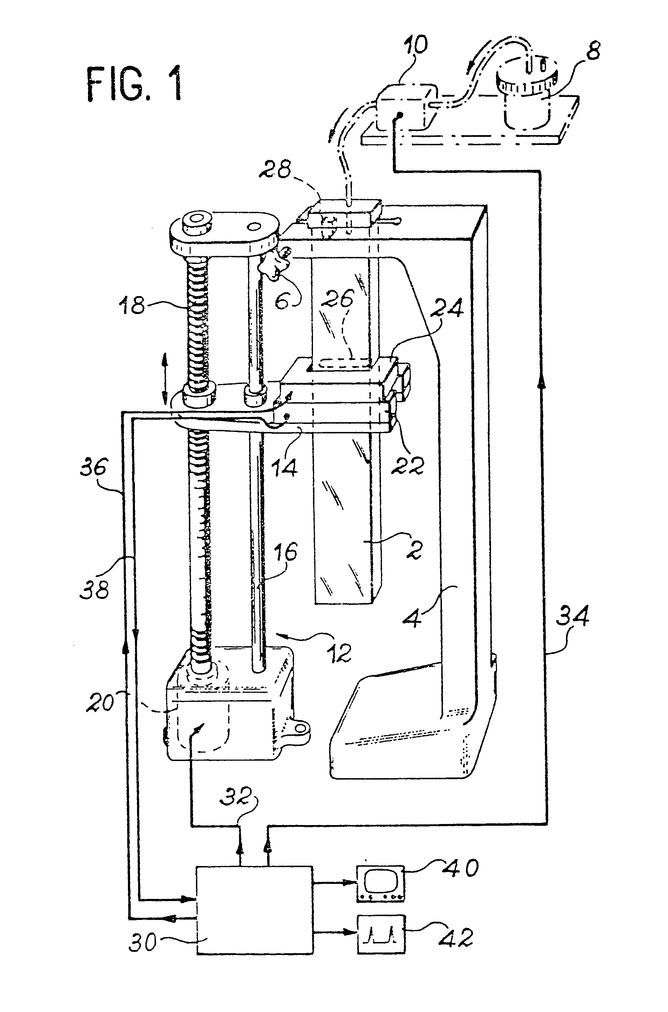

On a représenté sur la figure 1 un compteur de phases liquides à une cellule selon l'invention. Il comprend principalement une cellule 2 parallélépipédique contenant le mélange à analyser, un moyen de déplacement 12, un moyen de mesure optique 22, un moyen d'homogénéisation 24 et un moyen électronique 30 de commande et de traitement.

La cellule 2 est maintenue sur un support 4 par un moyen approprié tel qu'une vis 6. Près de ce support sont également disposées une source 8 d'un constituant liquide du mélange et une pompe volumétrique 10. Cette cellule a par exemple pour dimensions 10cm x 1cm x 2mm où la plus grande dimension est verticale.

Le moyen de déplacement 12 permet de déplacer un support 14 parallèlement à la cellule 2 et sur toute la hauteur de cette cellule. Il comprend un guide 16 pour limiter le déplacement du support à l'axe vertical et une vis 18 pilotée par un moteur pas à pas 20 pour déplacer verticalement ce support avec précision.

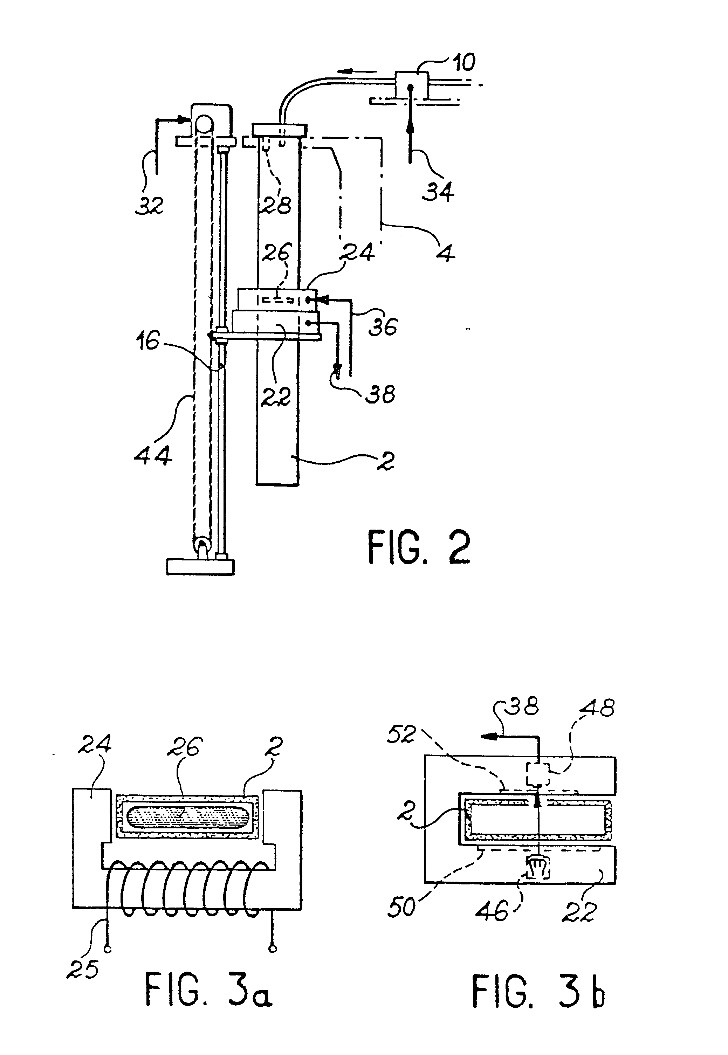

Sur le support 14 sont disposés un moyen de mesure optique 22 et un moyen d'homogénéisation 24 du mélange. Le moyen de mesure optique comporte une source lumineuse et un capteur optique disposés de part et d'autre de la cellule. La mesure par le capteur optique de la transmission du faisceau lumineux ayant traversé la cellule permet de déterminer si le mélange est en équilibre et de compter le nombre de phases de ce mélange. Le moyen d'homogénéisation 24 est par exemple de type magnétique. Il peut commander la rotation dans un plan vertical et/ou le déplacement vertical d'un agitateur 26 disposé dans la cellule. Pendant la mesure de la transmission optique, cet agitateur 26 est disposé dans une position de repos hors du mélange par exemple accroché à un aimant permanent 28. Ce moyen de mesure optique 22 et ce moyen d'homogénéisation 24 seront décrits en détail en référence aux figures 3a et 3b.

Le compteur de phases liquides comprend enfin un moyen électronique 30 de commande et de traitement. Il commande notamment par une connexion 32 le moteur 20 du moyen de déplacement 12, par une connexion 34 la mise en marche et le volume débité par la pompe volumétrique 10, par une voie 36 la mise en action du moyen d'homogénéisation 24. Il reçoit par une connexion 38 le signal délivré par le capteur optique du moyen de la mesure optique 22. Il déduit de ce signal le nombre de phases du mélange contenu dans la cellule. Le signal reçu du capteur optique peut être visualisé sur un écran 40 ou sur une sortie graphique 42. Ce moyen électronique 30 est par exemple un microordinateur de type DEC LSI 11.

On va maintenant expliquer le fonctionnement de ce compteur de phases liquides. A titre d'exemple, on va se référer à la construction d'un diagramme des phases d'un mélange partiellement liquide et transparent utilisé en récupération améliorée du pétrole. Un tel mélange comprend trois ou quatre constituants. Ces constituants sont un agent tensioactif, un hydrocarbure, un solvant, et éventuellement un agent cotensioactif souvent constitué d'un alcool.

Un mélange partiel comprenant tous les constituants sauf un ou plusieurs des constituants liquides est préparé manuellement et injecté dans la cellule. Dans le cas de la figure, seul un constituant liquide est absent du mélange. Il est mis dans la source 8. Ce constituant est par exemple le solvant.

Le moyen électronique 30 va d'abord commander une homogénéisation du mélange de la cellule, en activant le moyen d'homogénéisation 24 et/ou le moteur 20. Ensuite, le moyen électronique 30 commande la mise de l'agitateur 26 dans la position de repos 28, puis il attend l'équilibre thermodynamique du mélange.

Cet état thermodynamique du mélange est observé par les signaux reçus du capteur optique du moyen de mesure optique 22. Tant que l'équilibre n'est pas atteint, le mélange est trouble et donc la transmission optique faible.

Après un temps d'attente de décantation de durée prédéterminée, le moyen électronique 30 commande le déplacement du moyen de mesure 22 sur toute la hauteur de la cellule par l'intermédiaire du moteur 20. Le signal reçu sur la connexion 38, correspondant au signal lumineux reçu par le capteur optique pendant ce déplacement est alors traité, notamment numérisé et mémorisé, par le moyen électronique 30. Le nombre de phases est alors déduit de ce signal traité.

La visualisation sur l'écran 40 ou sur la sortie graphique 42 du signal reçu permet à un opérateur de contrôler que le nombre de phases relevé est correct. Ce contrôle peut se faire en temps différé, le signal étant mémorisé.

Lorsque le nombre de phases du mélange a été compté, le moyen électronique 30 commande par la connexion 34 l'injection d'un volume déterminé du constituant liquide de la source 8 dans la cellule 2. Celle-ci contient alors un nouveau mélange par la proportion de ces constituants. Celui-ci est traité comme le précédent.

Par injections successives au cours du temps de volumes déterminés du constituant liquide de la source 8, le compteur de l'invention permet de relever, sans intervention humaine, un grand nombre de points du diagramme des phases. Ce compteur de phases liquides permet donc un relevé rapide, précis et automatique d'un diagramme des phases d'un mélange partiellement liquide et transparent.

On a représenté sur la figure 2 une variante du mode de réalisation du compteur de phases liquides de la figure 1. Les éléments identiques à ceux de la figure 1 sont repérés par les mêmes références. Ce mode de réalisation diffère de celui de la figure précédente en ce qu'on a remplacé, dans le moyen de déplacement 12, la vis 18 par un câble 44.

Les figures 3a et 3b représentent, en coupe et en vue de dessus le moyen d'homogénéisation et le moyen de mesure optique.

Le moyen d'homogénéisation 24 est un électroaimant en forme de U excité par un courant parcourant l'enroulement électrique 25 et dont l'entrefer est occupé par la cellule 2. L'agitateur 26 est constitué d'un petit barreau aimanté, isolé chimiquement du milieu par une gaine polymérique, par exemple du polyéthylène. L'automatisation de l'homogénéisation par agitation est très délicate car il se forme facilement des mousses en présence d'agents tensioactifs. Pour éviter ceci, il faut utiliser cet agitateur magnétique avec prudence.

A cet effet, le moyen électronique 30 de commande peut commander le passage d'un courant électrique alternatif de fréquence faible ou rapide, dans l'enroulement 25 réalisant ainsi une mise en rotation lente ou rapide de l'agitateur 26, ou un courant électrique continu pour immobiliser l'agitateur. Il peut simultanément, en commandant le moteur 20, déplacer l'agitateur verticalement. En combinant les deux commandes, il peut notamment déplacer sans rotation l'agitateur, ce qui permet un léchage des parois de la cellule pour entraîner d'éventuelles gouttelettes de liquide ou de particules solides.

Le moyen de mesure optique 22 représenté sur la figure 3b comprend une source lumineuse 46 et un capteur optique 48 disposé en regard, ces deux éléments étant séparés par la cellule 2. Pour détecter l'interface entre deux phases liquides, il convient que la résolution du moyen de mesure optique soit importante. A cet effet, il comprend une fente 50 de 0,3 mm de hauteur disposée devant la source lumineuse 46 et une fente 52 de 0,07 mm de hauteur disposée devant le capteur optique 48. Ce moyen de mesure optique permet de distinguer le passage devant une interface entre deux phases avec une précision de l'ordre du dixième de millimètre.

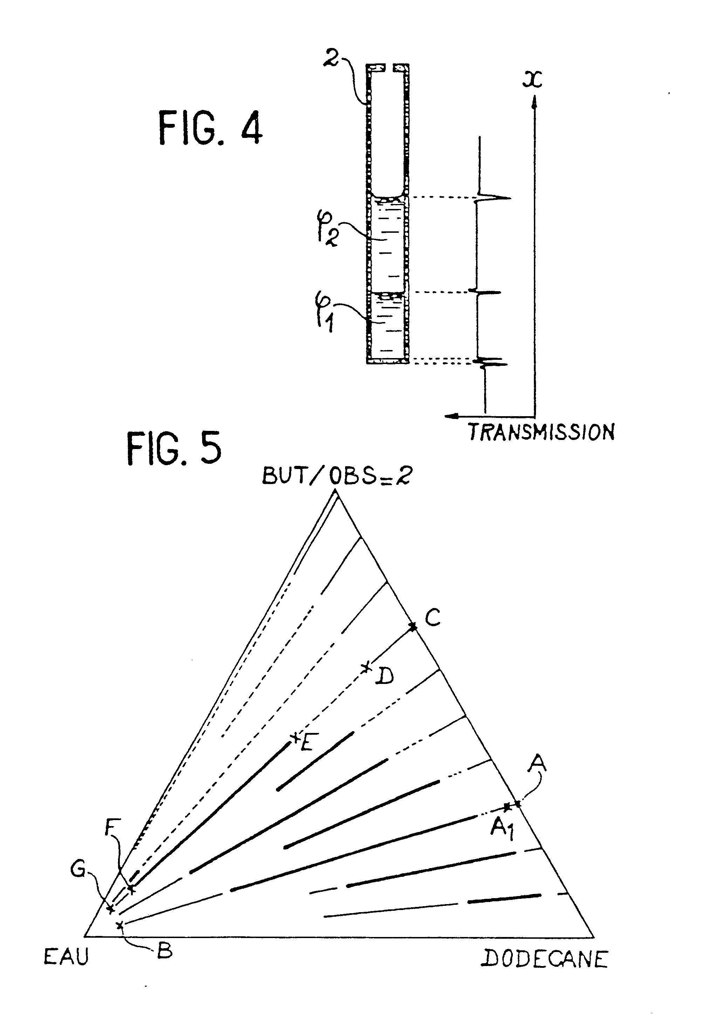

Le signal délivré par le capteur optique 48 est délivré par la connexion 38 au moyen du traitement 30. Ce signal est représenté sur la figure 4 en correspondance avec un mélange biphasique contenu dans la cellule 2. Le graphique représente la transmission en fonction de la position x. Les quatre pics correspondent respectivement à l'interface air-verre de la cellule, à l'interface verre de la cellule-phase $4_1$ du mélange, à l'interface des phases $4_1$ et $4_2$ du mélange et à l'interface de la phase $4_2$ et de l'air. Ce graphique est délivré sur un écran de visualisation ou sur une sortie graphique afin que l'utilisateur puisse effectuer un contrôle du nombre de phases détecté par le compteur.

La figure 5 représente un diagramme des phases obtenu avec un compteur selon l'invention. Le mélange étudié comprend un solvant (eau), un hydrocarbure (dodécane), un agent tensioactif (octyl-benzènesulfonate de sodium, en abrégé OBS) et un agent cotensioactif (butanol). Le rapport des proportions entre le butanol et l'OBS est égal à 2.

Le diagramme a été construit de la façon suivante: un mélange initial partiel, sans eau, de composition A a été préparé. Après avoir compté le nombre de phases de ce mélange, il lui a été ajouté un volume d'eau. La composition de ce nouveau mélange est représentée par le point $A_1$. Le nombre de phases de ce mélange a également été compté. Par injections successives de volumes d'eau, le compteur a ainsi compté le nombre de phases d'un grand nombre de mélanges situés sur le segment de droite AB.

En partant d'une composition initiale différente, telle que C, il détermine le nombre de phases d'un grand nombre de mélanges situés sur le segment de droite CG.

Le diagramme des phases est obtenu en traçant un nombre suffisant de segments de droite tels que AB et CG.

Sur le diagramme de la figure 5, les traits simples, tels que représentés entre les points C et D ou F et G correspondent à des mélanges polyphasiques contenant, après un temps d'attente déterminé consécutif à une agitation du mélange, une ou plusieurs phases opaques (présence d'un solide ou d'une émulsion).

Entre les points D et E, le mélange est monophasique et entre les points E et F, il est diphasique. Les mélanges utilisables en récupération améliorée du pétrole sont les mélanges monophasiques représentés en traits pointillés sur la figure.

7

Le compteur de la figure 1 permet d'obtenir ce diagramme avec une grande précision grâce à la dilution automatique du mélange dans la cellule et avec un gain de temps très important par rapport à l'art antérieur.

Cependant, ce compteur comporte un temps mort non négligeable dû au temps d'attente de décantation du mélange après homogénéisation. Ce temps mort du compteur peut être avantageusement utilisé pour traiter d'autres cellules, par exemple pour diluer un mélange ou pour compter le nombre de phases d'un mélange.

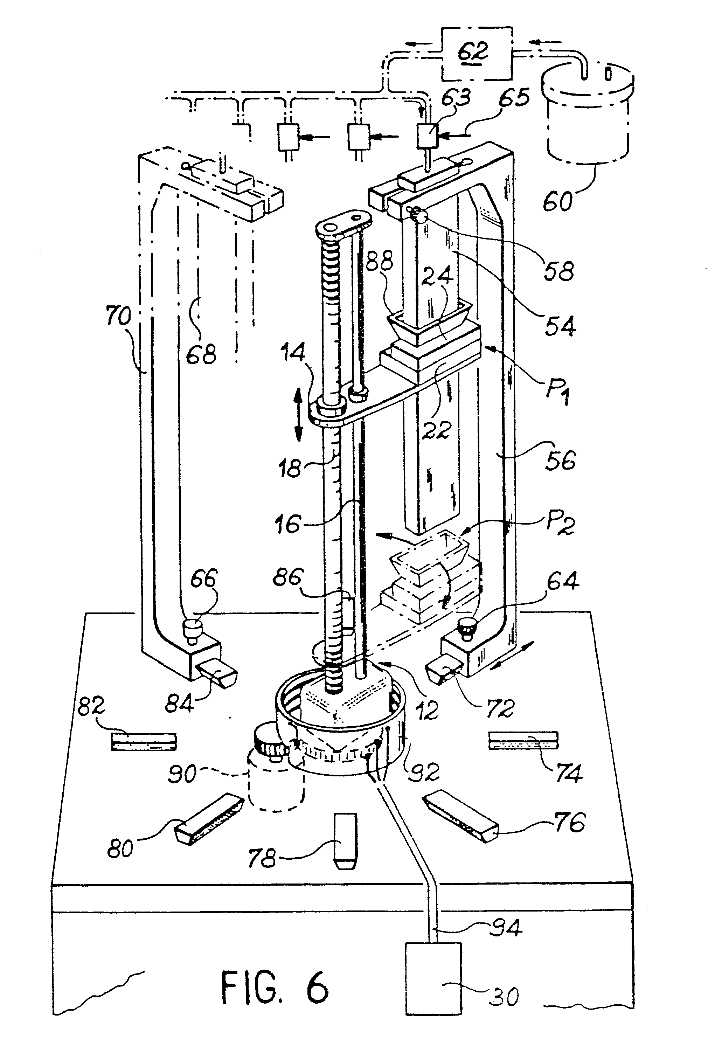

Le compteur de phases liquides à plusieurs cellules représenté sur la figure 6 permet de diminuer considérablement ce temps mort.

Les cellules sont disposées sur une couronne dont le centre coïncide avec l'axe de rotation du support 14. A titre d'exemple, le compteur de la figure 6 comporte huit cellules $C_1,...C_i,...C_8$. Une de ces cellules est représentée sur la figure sous la référence 54. Elle est fixée sur un support 56 par une vis 58. Le support 56 peut être déplacé manuellement dans une direction radiale sur une queue d'aronde 72. Une vis 64 assure la fixation du support dans la position choisie.

Une source 60 d'un constituant liquide du mélange et une pompe volumétrique 62 sont également prévues. Une électrovanne 63 commandée par une connexion 65 par le moyen électronique 30 relie la pompe 62 à la cellule 54. La pompe comprend en sortie plusieurs embranchements pour alimenter plusieurs cellules.

D'autres sources telles que 60 contenant d'autres constituants liquides peuvent être prévues.

Une autre cellule 68 et son support 70 sont esquissés. On a représenté également la queue d'aronde 84 guidant le déplacement du support 70 et la vis de serrage 66 correspondante. Les six autres queues d'arondes 74, 76, 78, 80, 82 et 86 indiquent la position des autres ensembles cellule-support.

Le compteur fonctionne de la manière suivante. Un mélange partiel initial est préparé manuellement dans chacune des cellules. Le constituant liquide à ajouter peut être contenu dans la source associée à chaque cellule et ajouté dans celle-ci à l'aide d'une pompe associée, soit contenu dans une source commune à plusieurs cellules, une pompe associée à la source délivrant dans chaque cellule le constituant liquide par l'intermédiaire d'une électrovanne commandée par le moyen électronique 30 et associée à chaque cellule.

Le moyen électronique 30 commande d'abord le positionnement du support 14 au droit de la cellule $C_1$ dans la position $P_2$. Ceci est réalisé par le moyen de positionnement 90. L'engagement en position $P_1$ du support dans la cellule est facilité par un cône d'entrée 88 disposé au-dessus du moyen d'homogénéisation 24. Ensuite, le moyen électronique 30 procède à l'homogénéisation du mélange de la cellule $C_1$ en activant le moyen d'homogénéisation 24 et le moyen de déplacement 12 par la voie 94. Celle-ci est reliée à une

8

couronne 92 qui comporte trois connexions électriques analogues aux connexions 32, 36 et 38 de la figure 1. Puis, l'agitateur est mis en position de repos et le support 14 est dégagé de la cellule. Pendant la décantation du mélange de la cellule $C_1$, le moyen électronique 30 procède successivement à l'homogénéisation des mélanges des cellules $C_2, C_3,...,C_8$.

Ainsi, le temps d'attente de retour à l'équilibre thermodynamique d'un mélange après homogénéisation, qui est un temps mort dans le compteur à une seule cellule, est ici utilisé activement.

Le gain de temps par rapport au compteur à une seule cellule est important. Il est optimum, et égal au nombre de cellules, si la durée de décantation du mélange d'une cellule dans un compteur à plusieurs cellules est égale à la durée nécessaire audit compteur pour homogénéiser le mélange de toutes les autres cellules.

## Revendications

1. Compteur de phases liquides d'un mélange partiellement liquide et transparent, le compteur comprenant:

- au moins une cellule (2) fixée sur un support (4), chaque cellule étant disposée verticalement et contenant un mélange partiellement liquide et transparent,
- un moyen d'homogénéisation (24) du mélange de chaque cellule,
- un moyen optique de mesure (22) de la transparence optique du mélange entre deux parois d'une cellule,
- un moyen de déplacement (12) pour déplacer le moyen d'homogénéisation et le moyen de mesure optique sur toute la hauteur d'une cellule parallèlement à celle-ci, et un moyen de positionnement (90) pour positionner lesdits moyens au droit de chaque cellule,
- un moyen électronique (30) de commande et de traitement délivrant des signaux pour commander les moyens de déplacement (12), de positionnement (90) et d'homogénéisation (24) et apte à déterminer le nombre de phases d'un mélange au vu des signaux reçus du moyen optique de mesure (22) et en ce qu'il comprend:
- une ou plusieurs sources (8) délivrant chacune un constituant liquide du mélange,
- au moins une pompe (10) par source,
- des électrovannes (63) commandées par le moyen électronique (30) pour permettre l'addition de chaque constituant liquide dans chaque cellule.

2. Compteur selon la revendication 1, *caractérisé* en ce que les cellules sont disposées sur une couronne circulaire dont le centre constitue l'axe du moyen de positionnement (90).

3. Compteur selon l'une quelconque des revendications 1 et 2 *caractérisé* en ce que le moyen de mesure optique comprend une source lumineuse et une photodiode en regard disposées de part et d'autre de la cellule.

4. Compteur selon l'une quelconque des revendications 1 à 3 *caractérisé* en ce que le moyen d'homogénéisation comprend un électroaimant et un barreau magnétique.

5. Compteur selon la revendication 4, *caractérisé* en ce qu'un aimant permanent est disposé dans la cellule ou à l'extérieur de celle-ci, au-dessus du mélange, constituant une position de repos pour le barreau magnétique.

## Patentansprüche

1. Flüssigkeitsphasenzähler einer teilweise flüssigen und durchsichtigen Mischung, wobei der Zähler aufweist:

wenigstens eine Zelle (2), die auf einem Träger (4) befestigt ist, wobei jede Zelle vertikal angeordnet ist und eine teilweise flüssige und durchsichtige Mischung enthält, eine Homogenisierungseinrichtung (24) der Mischung einer jeden Zelle, eine optische Meßeinrichtung (22) der optischen Durchsichtigkeit der Mischung zwischen zwei Seitenwänden einer Zelle, eine Verschiebeeinrichtung (12), um die Homogenisierungseinrichtung und die optische Meßeinrichtung auf der gesamten Höhe einer Zelle parallel zu dieser zu verschieben, und eine Positioniereinrichtung (90), um die genannten Einrichtungen im Bereich einer jeden Zelle zu positionieren, eine elektronische Einrichtung (30) der Steuerung und Verarbeitung, welche Signale für das Steuern der Verschiebeeinrichtung (12), der Positioniereinrichtung (90) und der Homogenisiereinrichtung (24) liefert und geeignet ist, die Anzahl der Phasen einer Mischung in Anbetracht der empfangenen Signale der optischen Meßeinrichtung (22) zu bestimmen und der aufweist:

eine oder mehrere Quellen (8), die jede einen flüssigen Bestandteil der Mischung liefert, wenigstens eine Pumpe (10) pro Quelle, Elektroventile (63), die durch die elektrische Einrichtung (30) betätigt werden, um den Zuschlag eines jeden flüssigen Bestandteiles in jeder Zelle zu erlauben.

2. Zähler nach Anspruch 1, *dadurch gekennzeichnet*, daß die Zellen auf einem kreisförmigen Kranz angeordnet sind, dessen Zentrum die Achse der Positioniereinrichtung (90) bildet.

3. Zähler nach Anspruch 1 oder 2, *dadurch gekennzeichnet*, daß die optische Meßeinrichtung eine Lichtquelle und eine Fotodiode aufweist, die gegenüber einerseits und andererseits der Zelle angeordnet ist.

4. Zähler nach einem der Ansprüche 1 bis 3, *dadurch gekennzeichnet*, daß die Homogenisierungseinrichtung einen Elektromagnet und einen magnetischen Gitterstab aufweist.

5. Zähler nach Anspruch 4, *dadurch gekennzeichnet*, daß ein Permanentmagnet in der Zelle oder an deren Äußerem oberhalb der Mischung angeordnet ist, was eine Ruhestellung für den magnetischen Gitterstab bildet.

## Claims

1. Liquid phase counter for a partly liquid and transparent mixture, incorporating:

- at least one cell (2) fixed to a support (4), each cell being arranged vertically and containing a partly liquid and transparent mixture,
- a means (24) for homogenizing the mixture of each cell,
- an optical means (22) for measuring the optical transparency of the mixture between two walls of a cell,
- a displacement means (12) for displacing the homogenization means and the measuring means over the entire height of a cell and parallel thereto, as well as a positioning means (90) for positioning said means to the right of each cell,
- an electronic control and processing means (30) supplying signals for controlling the displacement (12) and positioning (90) means and the homogenization means (24) and able to determine the number of phases of a mixture according to the signals received from the optical measuring means (22).

2. Counter according to claim 1, *characterized* in that the cells are located on a circular ring, whose centre constitutes the axis of the positioning means (90).

3. Counter according to either of the claims 1 and 2, *characterized* in that the optical measuring means comprises a lighting source and a facing photodiode arranged on either side of the cell.

4. Counter according to any one of the claims 1 to 3, *characterized* in that the homogenization means comprises an electromagnet and a magnetic bar.

5. Counter according to claim 4, *characterized* in that a permanent magnet is located in the cell or outside the same, above the mixture, constituting a rest position for the magnetic bar.

FIG. 1

FIG. 2

FIG. 3a

FIG. 3b

# FIG. 4

# FIG. 5

BUT/OBS=2

FIG. 6